# EUROPEAN PATENT SPECIFICATION

(11) **EP 3 787 598 B1**
(45) Date of publication and mention of the grant of the patent: **13.04.2022**
(21) Application number: 19721680.7
(22) Date of filing: 23.04.2019
(51) Int. Cl.: A61K 9/00, A61K 31/137, A61P 11/08, A61K 45/06

(54) **PHARMACEUTICAL COMPOSITION COMPRISING SALBUTAMOL**
SALBUTAMOL ENTHALTENDE PHARMAZEUTISCHE ZUSAMMENSETZUNG
COMPOSITION PHARMACEUTIQUE COMPRENANT DU SALBUTAMOL

(30) Priority: 30.04.2018 GB 201807053
(43) Date of publication of application: 10.03.2021
(73) Proprietor: Mexichem Fluor S.A. de C.V., C.P. 78395 (MX)
(72) Inventor: NOAKES, Timothy James, Mold, Flintshire CH7 5JF (GB); CORR, Stuart, Warrington, Cheshire WA4 5DH (GB)
(74) Representative: Potter Clarkson
(86) International application number: PCT/GB2019/051136
(87) International publication number: WO 2019/211578

(56) References cited:
- WO-A1-2018/051133
- WO-A2-2010/052466
- GB-A- 2 554 090
- GB-A- 2 554 091
- US-A1- 2004 184 994
- US-A1- 2007 183 982
- US-A1- 2014 286 877
- US-A1- 2016 058 714
- US-A1- 2017 165 367

## Description

The present invention relates to the delivery of drug formulations from a medical device, such as a metered dose inhaler (MDI), using a propellant comprising 1,1-difluoroethane (HFA-152a). More particularly, the present invention relates to pharmaceutical compositions comprising HFA-152a propellant and a drug formulation which is dissolved or suspended in the propellant and to medical devices containing those compositions. The pharmaceutical compositions of the invention are particularly suited for delivery from a pressurised aerosol container using a metered dose inhaler (MDI).

MDIs are the most significant type of inhalation drug delivery system and are well known to those skilled in the art. They are designed to deliver, on demand, a discrete and accurate amount of a drug to the respiratory tract of a patient using a liquefied propellant in which the drug is dissolved, suspended or dispersed. The design and operation of MDIs is described in many standard textbooks and in the patent literature. They all comprise a pressurised container that holds the drug formulation, a nozzle and a valve assembly that is capable of dispensing a controlled quantity of the drug through the nozzle when it is activated. The nozzle and valve assembly are typically located in a housing that is equipped with a mouth piece. The drug formulation will comprise a propellant, in which the drug is dissolved, suspended or dispersed, and may contain other materials such as polar excipients, surfactants and preservatives.

In order for a propellant to function satisfactorily in MDIs, it needs to have a number of properties. These include an appropriate boiling point and vapour pressure so that it can be liquefied in a closed container at room temperature but develop a high enough pressure when the MDI is activated to deliver the drug as an atomised formulation even at low ambient temperatures. Further, the propellant should be of low acute and chronic toxicity and have a high cardiac sensitisation threshold. It should have a high degree of chemical stability in contact with the drug, the container and the metallic and non-metallic components of the MDI device, and have a low propensity to extract low molecular weight substances from any elastomeric materials in the MDI device. The propellant should also be capable of maintaining the drug in a homogeneous solution, in a stable suspension or in a stable dispersion for a sufficient time to permit reproducible delivery of the drug in use. When the drug is in suspension in the propellant, the density of the liquid propellant is desirably similar to that of the solid drug in order to avoid rapid sinking or floating of the drug particles in the liquid. Finally, the propellant should not present a significant flammability risk to the patient in use. In particular, it should form a non-flammable or low flammability mixture when mixed with air in the respiratory tract.

Dichlorodifluoromethane (R-12) possesses a suitable combination of properties and was for many years the most widely used MDI propellant, often blended with trichlorofluoromethane (R-11). Due to international concern that fully and partially halogenated chlorofluorocarbons (CFCs), such as dichlorodifluoromethane and trichlorofluoromethane, were damaging the earth's protective ozone layer, many countries entered into an agreement, the Montreal Protocol, stipulating that their manufacture and use should be severely restricted and eventually phased out completely. Dichlorodifluoromethane and trichlorofluoromethane were phased out for refrigeration use in the 1990's, but are still used in small quantities in the MDI sector as a result of an essential use exemption in the Montreal Protocol.

1,1,1,2-tetrafluoroethane (HFA-134a) was introduced as a replacement refrigerant and MDI propellant for R-12. 1,1,1,2,3,3,3-heptafluoropropane (HFA-227ea) was also introduced as a replacement propellant for dichlorotetrafluoroethane (R-114) in the MDI sector and is sometimes used alone or blended with HFA -134a for this application.

Although HFA-134a and HFA-227ea have low ozone depletion potentials (ODPs), they have global warming potentials (GWPs), 1430 and 3220 respectively, which are now considered to be too high by some regulatory bodies, especially for dispersive uses when they are released into the atmosphere.

One industrial area that has received particular attention recently has been the automotive air-conditioning sector where the use of HFA-134a has come under regulatory control as a result of the European Mobile Air Conditioning Directive (2006/40/EC). Industry has developed a number of possible alternatives to HFA-134a in automotive air conditioning and other applications that have a low greenhouse warming potential (GWP) as well as a low ozone depletion potential (ODP). Many of these alternatives include hydrofluoropropenes, especially the tetrafluoropropenes, such as 2,3,3,3-tetrafluoropropene (HFO-1234yf) and 1,3,3,3-tetrafluoropropene (HFO-1234ze).

Although the proposed alternatives to HFA-134a have a low GWP, the toxicological status of many of the components, such as certain of the fluoropropenes, is unclear and they are unlikely to be acceptable for use in the MDI sector for many years, if at all.

Salbutamol is a short-acting bronchodilator used in the treatment and control of a number of respiratory-related disorders, but particularly asthma and chronic obstructive pulmonary disease (COPD). The drug is conveniently delivered using a MDI.

WO2010052466 A2 discloses a pharmaceutical aerosol composition wherein the active agent may be salbutamol and the HFA propellant is HFA-134a, HFA-227 or a mixture thereof.

There is a need for a pharmaceutical composition containing salbutamol which can, be delivered using a MDI and that uses a propellant having a reduced GWP in comparison with HFA-134a and HFA-227ea. There is also a need for a salbutamol-containing pharmaceutical composition which exhibits improved stability.

We have found that a propellant comprising 1,1-difluoroethane (HFA-152a) can be used to successfully deliver salbutamol-containing drug formulations using a MDI. These formulations can exhibit improved chemical stability, particularly where the formulations contain low amounts of water, reduced GWP, good compatibility with standard uncoated aluminium cans as well as good compatibility with standard valves and seals.

According to a first aspect of the present invention, there is provided a pharmaceutical composition, e.g. a pharmaceutical suspension or a pharmaceutical solution, said composition comprising:
(i) a drug component comprising salbutamol base; and
(ii) a propellant component at least 90 weight % of which is 1,1-difluoroethane (HFA-152a).

The pharmaceutical composition of the first aspect of the invention typically contains less than 1000 ppm, e.g. less than 500 ppm, of water based on the total weight of the pharmaceutical composition. The improved chemical stability can be observed, in particular, when the pharmaceutical composition contains less than 100 ppm, preferably less than 50 ppm, more preferably less than 10 ppm and particularly less than 5 ppm of water based on the total weight of the pharmaceutical composition. In referring to the water content of the pharmaceutical composition, we are referring to the content of free water in the composition and not any water that happens to be present in any hydrated drug compounds that may be used as part of the drug component. In an especially preferred embodiment, the pharmaceutical composition is water-free. Alternatively, the pharmaceutical composition of the first aspect may contain greater than 0.5 ppm of water, e.g. greater than 1 ppm, but less than the amounts discussed above, as it can in practice be difficult to remove all the water from the composition and then retain it in such a water-free state.

Accordingly, a preferred embodiment of the first aspect of the present invention provides a pharmaceutical composition, e.g. a pharmaceutical suspension or a pharmaceutical solution, said composition comprising:
(i) a drug component comprising salbutamol base; and
(ii) a propellant component at least 90 weight % of which is 1,1-difluoroethane (HFA-152a),
   wherein the composition contains less than 100 ppm, preferably less than 50 ppm, more preferably less than 10 ppm and especially less than 5 ppm of water based on the total weight of the pharmaceutical composition.

In a preferred embodiment, the pharmaceutical composition of the first aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the first aspect may contain greater than 0.5 ppm of oxygen, e.g. 1 ppm or greater, but less than the amounts discussed above, as it can in practice be difficult to retain the composition in an oxygen-free state. Low oxygen contents are preferred because they can tend to reduce the degradation of the drug compounds resulting in a composition with higher chemical stability.

Accordingly, a preferred embodiment of the first aspect of the present invention provides a pharmaceutical composition, e.g. a pharmaceutical suspension or a pharmaceutical solution, said composition comprising:
(i) a drug component comprising salbutamol base; and
(ii) a propellant component at least 90 weight % of which is 1,1-difluoroethane (HFA-152a),
   wherein the composition contains less than 1000 ppm, preferably less than 500 ppm, more preferably less than 100 ppm and especially less than 50 ppm of oxygen based on the total weight of the pharmaceutical composition.

The pharmaceutical composition of the present invention is suitable for delivery to the respiratory tract using a metered dose inhaler (MDI).

The salbutamol that is included in the pharmaceutical composition of the invention in all aspects and embodiments disclosed herein is salbutamol base, i.e. the term is intended to exclude all pharmaceutically acceptable derivatives, e.g. salts and prodrugs, of salbutamol. The salbutamol is preferably in a micronized form. Further, the pharmaceutical composition of the invention in all aspects and embodiments disclosed herein is preferably free of perforated microstructures.

The salbutamol may be dispersed or suspended in the propellant. The drug particles in such suspensions preferably have a diameter of less than 100 microns, e.g. less than 50 microns. However, in an alternative embodiment the pharmaceutical compositions of the invention are solutions with the salbutamol dissolved in the propellant, e.g. with the assistance of a polar excipient, such as ethanol.

The amount of the drug component in the pharmaceutical composition of the first aspect of the present invention will typically be in the range of from 0.01 to 2.5 weight % based on the total weight of the pharmaceutical composition. Preferably, the drug component will comprise from 0.01 to 2.0 weight %, more preferably from 0.05 to 2.0 weight % and especially from 0.05 to 1.5 weight % of the total weight of the pharmaceutical composition. The drug component may consist essentially of or consist entirely of salbutamol base. By the term "consists essentially of", we mean that at least 98 weight %, more preferably at least 99 weight % and especially at least 99.9 weight % of the drug component consists of salbutamol. Alternatively, the drug component may contain other drugs, such as at least one corticosteroid and/or least one long acting muscarinic antagonist (LAMA).

The propellant component in the pharmaceutical composition of the first aspect of the present invention comprises 1,1-difluoroethane (HFA-152a). Thus, we do not exclude the possibility that the propellant component may include other propellant compounds in addition to the HFA-152a. For example, the propellant component may additionally comprise one or more additional hydrofluorocarbon or hydrocarbon propellant compounds, e.g. selected from HFA-227ea, HFA-134a, difluoromethane (HFA-32), propane, butane, isobutane and dimethyl ether. The preferred additional propellants are HFA-227ea and HFA-134a.

If an additional propellant compound is included, such as HFA-134a or HFA-227ea, the HFA-152a will constitute at least 90 weight %, e.g. from 90 to 99 weight %, of the propellant component. Preferably, the HFA-152a will constitute at least 95 weight %, e.g. from 95 to 99 weight %, and more preferably at least 99 weight % of the propellant component.

In a preferred embodiment, the propellant component has a global warming potential (GWP) of less than 250, more preferably less than 200 and still more preferably less than 150.

In an especially preferred embodiment, the propellant component consists entirely of HFA-152a so that the pharmaceutical composition of the invention comprises HFA-152a as the sole propellant. By the term "consists entirely of" we do not, of course, exclude the presence of minor amounts, e.g. up to a few hundred parts per million, of impurities that may be present following the process that is used to make the HFA-152a providing that they do not affect the suitability of the propellant in medical applications. Preferably the HFA-152a propellant will contain no more than 10 ppm, e.g. from 0.5 to 10 ppm, more preferably no more than 5 ppm, e.g. from 1 to 5 ppm, of unsaturated impurities, such as vinyl fluoride, vinyl chloride, vinylidene fluoride and chloro-fluoro ethylene compounds.

The amount of propellant component in the pharmaceutical composition of the invention will vary depending on the amounts of the drugs and other components in the pharmaceutical composition. However, whatever drugs and other components are included in the pharmaceutical composition the propellant component will make up the remainder and constitute the bulk of the composition overall. Typically, the propellant component will comprise from 74.0 to 99.99 weight % of the total weight of the pharmaceutical composition. Preferably, the propellant component will comprise from 82.5 to 99.99 weight %, more preferably from 87.5 to 99.99 weight % and especially from 92.5 to 99.95 weight % of the total weight of the pharmaceutical composition.

In one embodiment, the pharmaceutical composition of the first aspect of the present invention consists essentially of and more preferably consists entirely of the two components (i) and (ii) listed above. By the term "consists essentially of", we mean that at least 98 weight %, more preferably at least 99 weight % and especially at least 99.9 weight % of the pharmaceutical composition consists of the two listed components.

In another preferred embodiment, the pharmaceutical composition of the first aspect of the present invention additionally includes a polar excipient, such as ethanol. Polar excipients have been used previously in pharmaceutical compositions for treating respiratory disorders that are delivered using metered dose inhalers (MDIs). They are also referred to as solvents, co-solvents, carrier solvents and adjuvants. Their inclusion can serve to solubilise the surfactant or the drug in the propellant and/or inhibit deposition of drug particles on the surfaces of the metered dose inhaler that are contacted by the pharmaceutical composition as it passes from the container in which it is stored to the nozzle outlet. They are also used as bulking agents in two-stage filling processes where the drug is mixed with a suitable polar excipient. The most commonly used polar excipient is ethanol. If a polar excipient is used, it will typically be present in an amount of from 0.5 to 25.0 % by weight, preferably in an amount of from 1 to 15 % by weight based on the total weight of the pharmaceutical composition.

In one preferred embodiment, the pharmaceutical composition of the present invention comprises ethanol.

The pharmaceutical composition of the first aspect of the present invention may also include a surfactant component comprising at least one surfactant compound. Where the pharmaceutical composition is a suspension, the surfactant component is preferably not present as a surface coating on the drug particles. Drug particles with such surface coatings are prepared by pre-coating the drug particles with the surfactant component prior to mixing with the propellant component.

Surfactant compounds of the type that have been in use hitherto in pharmaceutical formulations for MDIs may be used in the pharmaceutical compositions of the present invention. Preferred surfactants are selected from polyvinylpyrrolidone, polyethylene glycol surfactants, oleic acid and lecithin. By the term oleic acid, we are not necessarily referring to pure (9Z)-octadec-9-enoic acid. When sold for surfactant use in medical applications, oleic acid is typically a mixture of several fatty acids, with (9Z)-octadec-9-enoic acid being the predominant fatty acid, e.g. present in an amount of at least 65 weight % based on the total weight of the surfactant.

If a surfactant component is included, it is preferably free of fluorinated surfactant compounds. In another embodiment, the surfactant component is free of surfactant compounds selected from C₈₋₁₆ fatty acids or salts, bile salts, phospholipids and alkyl saccharides.

In a preferred embodiment, the surfactant component consists essentially of and still more preferably consists entirely of at least one surfactant compound selected from polyvinylpyrrolidone, polyethylene glycols, oleic acid and lecithin. In a particularly preferred embodiment, the surfactant component consists essentially of and still more preferably consists entirely of at least one surfactant compound selected from polyvinylpyrrolidone and polyethylene glycols. By the term "consists essentially of", we mean that at least 95 weight %, more preferably at least 98 weight % and especially at least 99 weight % of the surfactant component is composed of the listed surfactants.

If a surfactant component is used, it will typically be present in an amount of from 0.1 to 2.5 % by weight, preferably in an amount of from 0.1 to 2.0 % by weight and more preferably in an amount of from 0.2 to 1.5 % by weight based on the total weight of the pharmaceutical composition.

The pharmaceutical composition of the invention may also include a long acting muscarinic antagonist (LAMA). Any of the long acting muscarinic antagonists that have been in use hitherto for treating chronic obstructive pulmonary diseases and that can be delivered using a MDI can be used in the pharmaceutical compositions of the present invention. Suitable long acting muscarinic antagonists include umeclidinium, ipratropium, tiotropium, aclidinium and the pharmaceutically acceptable derivatives thereof, especially the pharmaceutically acceptable salts thereof, as well as pharmaceutically acceptable salts of glycopyrrolate (also known as glycopyrronium). Glycopyrrolate is a quaternary ammonium salt. Suitable pharmaceutically acceptable counter ions include, for example, fluoride, chloride, bromide, iodide, nitrate, sulfate, phosphate, formate, acetate, trifluoroacetate, propionate, butyrate, lactate, citrate, tartrate, malate, maleate, succinate, benzoate, p-chlorobenzoate, diphenyl-acetate or triphenylacetate, o-hydroxybenzoate, p-hydroxybenzoate, 1- hydroxynaphthalene-2-carboxylate, 3-hydroxynaphthalene-2-carboxylate, methanesulfonate and benzenesulfonate. A preferred glycopyrrolate salt is the bromide salt, also known as glycopyrronium bromide.

Preferred long-acting muscarinic antagonists are selected from tiotropium, ipratropium and their pharmaceutically acceptable salts, especially tiotropium bromide and ipratropium bromide.

Accordingly, a second aspect of the present invention provides a pharmaceutical composition, e.g. a pharmaceutical suspension or a pharmaceutical solution, said composition comprising:
(i) a drug component comprising salbutamol base and at least one long acting muscarinic antagonist, particularly at least one long acting muscarinic antagonist selected from tiotropium, ipratropium and their pharmaceutically acceptable salts, especially tiotropium bromide and ipratropium bromide; and
(ii) a propellant component at least 90 weight % of which is 1,1-difluoroethane (HFA-152a).

The pharmaceutical composition of the second aspect of the invention typically contains less than 1000 ppm, e.g. less than 500 ppm of water based on the total weight of the pharmaceutical composition. Preferably, the pharmaceutical composition of the second aspect of the invention contains less than 100 ppm, more preferably less than 50 ppm, particularly less than 10 ppm and especially less than 5 ppm of water based on the total weight of the pharmaceutical composition.

It has been found that small amounts of water alongside the use of HFA-152a as the propellant can result in a pharmaceutical composition with improved chemical stability. In referring to the water content of the pharmaceutical composition, we are referring to the content of free water in the composition and not any water that happens to be present in any hydrated drug compounds that may be used as part of the drug component. In an especially preferred embodiment, the pharmaceutical composition of the second aspect of the present invention is water-free. Alternatively, the pharmaceutical composition of the second aspect may contain greater than 0.5 ppm of water, e.g. greater than 1 ppm, but less than the amounts discussed above, as it can in practice be difficult to remove all the water from the composition and then retain it in such a water-free state.

In a preferred embodiment, the pharmaceutical composition of the second aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the second aspect may contain greater than 0.5 ppm of oxygen, e.g. 1 ppm or greater, but less than the amounts discussed above, as it can in practice be difficult to retain the composition in an oxygen-free state. Low oxygen contents are preferred because they can tend to reduce the degradation of the drug compounds resulting in a composition with higher chemical stability.

Typical and preferred amounts of the drug component and the propellant component in the pharmaceutical composition of the second aspect of the present invention and suitable, typical and preferred compositions for the propellant component are as discussed above for the pharmaceutical composition of the first aspect of the invention. The drug component may consist essentially of or consist entirely of salbutamol and the at least one long acting muscarinic antagonist. By the term "consists essentially of", we mean that at least 98 weight %, more preferably at least 99 weight % and especially at least 99.9 weight % of the drug component consists of salbutamol and the at least one long acting muscarinic antagonist.

In one embodiment, the pharmaceutical composition of the second aspect of the present invention consists essentially of and more preferably consists entirely of the two components (i) and (ii) listed above. By the term "consists essentially of", we mean that at least 98 weight %, more preferably at least 99 weight % and especially at least 99.9 weight % of the pharmaceutical composition consists of the two listed components.

In another embodiment, the pharmaceutical composition of the second aspect of the invention may contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In an especially preferred embodiment of the second aspect of the invention, the drug component comprises salbutamol and at least one long acting muscarinic antagonist selected from tiotropium, ipratropium, tiotropium bromide and ipratropium bromide. Preferably, the salbutamol and the at least one selected long acting muscarinic antagonist are the only pharmaceutical actives in the pharmaceutical composition of the second aspect of the invention.

The pharmaceutical composition of the invention may also include a corticosteroid. Any of the corticosteroids that have been in use hitherto for treating asthma and chronic obstructive pulmonary diseases and that can be delivered using a MDI can be used in the pharmaceutical compositions of the present invention. Suitable corticosteroids include budesonide, mometasone, beclomethasone and fluticasone as well as their pharmaceutically acceptable derivatives, such as their pharmaceutically acceptable salts and esters. Preferred compounds include beclomethasone and beclomethasone dipropionate.

Accordingly, a third aspect of the present invention provides a pharmaceutical composition, e.g. a pharmaceutical suspension or a pharmaceutical solution, said composition comprising:
(i) a drug component comprising salbutamol base and at least one corticosteroid, particularly at least one corticosteroid selected from fluticasone, budesonide, mometasone and beclomethasone and the pharmaceutically acceptable derivatives thereof, especially beclomethasone and beclomethasone dipropionate; and
(ii) a propellant component at least 90 weight % of which is 1,1-difluoroethane (HFA-152a).

The pharmaceutical composition of the third aspect of the invention typically contains less than 1000 ppm, e.g. less than 500 ppm of water based on the total weight of the pharmaceutical composition. Preferably, the pharmaceutical composition of the third aspect of the invention contains less than 100 ppm, more preferably less than 50 ppm, particularly less than 10 ppm and especially less than 5 ppm of water based on the total weight of the pharmaceutical composition. It has been found that small amounts of water alongside the use of HFA-152a as the propellant can result in a pharmaceutical composition with improved chemical stability. In referring to the water content of the pharmaceutical composition, we are referring to the content of free water in the composition and not any water that happens to be present in any hydrated drug compounds that may be used as part of the drug component. In an especially preferred embodiment, the pharmaceutical composition of the third aspect of the present invention is water-free. Alternatively, the pharmaceutical composition of the third aspect may contain greater than 0.5 ppm of water, e.g. greater than 1 ppm, but less than the amounts discussed above, as it can in practice be difficult to remove all the water from the composition and then retain it in such a water-free state.

In a preferred embodiment, the pharmaceutical composition of the third aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the third aspect may contain greater than 0.5 ppm of oxygen, e.g. 1 ppm or greater, but less than the amounts discussed above, as it can in practice be difficult to retain the composition in an oxygen-free state. Low oxygen contents are preferred because they can tend to reduce the degradation of the drug compounds resulting in a composition with higher chemical stability.

Typical and preferred amounts of the drug component and the propellant component in the pharmaceutical composition of the third aspect of the present invention and suitable, typical and preferred compositions for the propellant component are as discussed above for the pharmaceutical composition of the first aspect of the invention. The drug component may consist essentially of or consist entirely of salbutamol and the at least one corticosteroid. By the term "consists essentially of", we mean that at least 98 weight %, more preferably at least 99 weight % and especially at least 99.9 weight % of the drug component consists of salbutamol and the at least one corticosteroid.

In one embodiment, the pharmaceutical composition of the third aspect of the present invention consists essentially of and more preferably consists entirely of the two components (i) and (ii) listed above. By the term "consists essentially of", we mean that at least 98 weight %, more preferably at least 99 weight % and especially at least 99.9 weight % of the pharmaceutical composition consists of the two listed components.

In another embodiment, the pharmaceutical composition of the third aspect of the invention may contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In an especially preferred embodiment of the third aspect of the invention, the drug component comprises salbutamol and at least one corticosteroid selected from beclomethasone and beclomethasone dipropionate. Preferably, the salbutamol and the beclomethasone and/or beclomethasone dipropionate are the only pharmaceutical actives in the pharmaceutical composition of the third aspect of the invention.

The pharmaceutical composition of the invention may also include a long acting muscarinic antagonist (LAMA) and a corticosteroid. Any of the long acting muscarinic antagonists and corticosteroids that have been in use hitherto for treating asthma and chronic obstructive pulmonary diseases and that can be delivered using a MDI can be used in the pharmaceutical compositions of the present invention. Suitable and preferred long acting muscarinic antagonists are as discussed above for the second aspect of the invention. Suitable and preferred corticosteroids are as discussed above for the third aspect of the present invention.

Accordingly, a fourth aspect of the present invention provides a pharmaceutical composition, e.g. a pharmaceutical suspension or a pharmaceutical solution, said composition comprising:
(i) a drug component comprising salbutamol base, at least one long acting muscarinic antagonist, particularly at least one long acting muscarinic antagonist selected from tiotropium, ipratropium and their pharmaceutically acceptable salts, especially tiotropium bromide and ipratropium bromide, and at least one corticosteroid, particularly at least one corticosteroid selected from fluticasone, budesonide, mometasone and beclomethasone and the pharmaceutically acceptable derivatives thereof, especially beclomethasone and beclomethasone dipropionate; and
(ii) a propellant component at least 90 weight % of which is 1,1-difluoroethane (HFA-152a).

The pharmaceutical composition of the fourth aspect of the invention typically contains less than 1000 ppm, e.g. less than 500 ppm of water based on the total weight of the pharmaceutical composition. Preferably, the pharmaceutical composition of the fourth aspect of the present invention contains less than 100 ppm, more preferably less than 50 ppm, particularly less than 10 ppm and especially less than 5 ppm of water based on the total weight of the pharmaceutical composition. It has been found that small amounts of water alongside the use of HFA-152a as the propellant can result in a pharmaceutical composition with improved chemical stability. In referring to the water content of the pharmaceutical composition, we are referring to the content of free water in the composition and not any water that happens to be present in any hydrated drug compounds that may be used as part of the drug component. In an especially preferred embodiment, the pharmaceutical composition of the fourth aspect of the present invention is water-free. Alternatively, the pharmaceutical composition of the fourth aspect may contain greater than 0.5 ppm of water, e.g. greater than 1 ppm, but less than the amounts discussed above, as it can in practice be difficult to remove all the water from the composition and then retain it in such a water-free state.

In a preferred embodiment, the pharmaceutical composition of the fourth aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the fourth aspect may contain greater than 0.5 ppm of oxygen, e.g. 1 ppm or greater, but less than the amounts discussed above, as it can in practice be difficult to retain the composition in an oxygen-free state. Low oxygen contents are preferred because they can tend to reduce the degradation of the drug compounds resulting in a composition with higher chemical stability.

Typical and preferred amounts of the drug component and the propellant component in the pharmaceutical composition of the fourth aspect of the present invention and suitable, typical and preferred compositions for the propellant component are as discussed above for the pharmaceutical composition of the first aspect of the invention. The drug component may consist essentially of or consist entirely of salbutamol, the at least one long acting muscarinic antagonist (LAMA) and the at least one corticosteroid. By the term "consists essentially of", we mean that at least 98 weight %, more preferably at least 99 weight % and especially at least 99.9 weight % of the drug component consists of salbutamol, the at least one long acting muscarinic antagonist (LAMA) and the at least one corticosteroid.

In one embodiment, the pharmaceutical composition of the fourth aspect of the present invention consists essentially of and more preferably consists entirely of the two components (i) and (ii) listed above. By the term "consists essentially of", we mean that at least 98 weight %, more preferably at least 99 weight % and especially at least 99.9 weight % of the pharmaceutical composition consists of the two listed components.

In another embodiment, the pharmaceutical composition of the fourth aspect of the invention may contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In an especially preferred embodiment of the fourth aspect of the invention, the drug component comprises salbutamol, at least one long acting muscarinic antagonist selected from tiotropium, ipratropium, tiotropium bromide and ipratropium bromide, and at least one corticosteroid selected from beclomethasone and beclomethasone dipropionate. Preferably, the salbutamol, the at least one selected long acting muscarinic antagonist and the beclomethasone and/or beclomethasone dipropionate are the only pharmaceutical actives in the pharmaceutical composition of the fourth aspect of the invention.

Any of the pharmaceutical compositions of the invention may include an acid stabiliser. Suitable acid stabilisers include organic acids, such as citric acid, oleic acid and ethanoic acid, and mineral acids, such as hydrochloric acid, nitric acid and phosphoric acid. Where an acid stabiliser is used, mineral acids are preferred. Alternatively, the pharmaceutical compositions of the invention may be free of acid stabilisers.

It has been found that the use of propellants comprising 1,1-difluoroethane (HFA-152a) in pharmaceutical compositions containing salbutamol and the propellant can unexpectedly improve the chemical stability of the salbutamol compared to the stability it exhibits in formulations containing HFA-134a as the propellant.

Accordingly, in a fifth aspect of the present invention there is provided a method of improving the stability of a pharmaceutical composition comprising a propellant component and a drug component comprising salbutamol base, said method comprising using a propellant component at least 90 weight % of which is 1,1-difluoroethane (HFA-152a).

The pharmaceutical composition in the stabilisation method of the fifth aspect of the present invention may be a suspension or a solution, but is typically a suspension.

The improved chemical stability can result, in particular, when the pharmaceutical composition contains less than 1000 ppm, preferably less than 500 ppm, more preferably less than 100 ppm, still more preferably less than 50 ppm, particularly less than 10 ppm and especially less than 5 ppm of water based on the total weight of the pharmaceutical composition. In referring to the water content of the pharmaceutical composition, we are referring to the content of free water in the composition and not any water that happens to be present in any hydrated drug compounds that may be used as part of the drug component. In an especially preferred embodiment, the pharmaceutical composition is water-free. Alternatively, the pharmaceutical composition recited in the fifth aspect of the present invention may contain greater than 0.5 ppm of water, e.g. greater than 1 ppm, but less than the amounts discussed above, as it can in practice be difficult to remove all the water from the composition and then retain it in such a water-free state.

Accordingly, in a preferred embodiment of the fifth aspect of the present invention there is provided a method of improving the stability of a pharmaceutical composition comprising a propellant component and a drug component comprising salbutamol, said method comprising using a propellant component comprising 1,1-difluoroethane (HFA-152a) and selecting the components and conditions for the preparation of the pharmaceutical composition to maintain the water content of the pharmaceutical composition below 1000 ppm, e.g. below 500 ppm, preferably below 100 ppm, more preferably below 50 ppm, still more preferably below 10 ppm and particularly below 5 ppm based on the total weight of the pharmaceutical composition.

In practice, preparing a pharmaceutical composition with the low water levels recited above involves using a propellant component with a suitably low water content, as it is usually the largest mass item in the finished device, and then preparing the pharmaceutical composition under suitably dry conditions, e.g. in a dry nitrogen atmosphere. Preparing pharmaceutical compositions under dry conditions is well known and the techniques involved are well understood by those skilled in the art. Other steps to obtain a low water content in the finished device include drying and storing the can and valve components in a moisture-controlled atmosphere, e.g. dry nitrogen or air, prior to and during device assembly. If the pharmaceutical composition contains a significant amount of ethanol, then it may also be important to control the water content of the ethanol as well as the propellant, e.g. by drying to reduce the water content to suitably low levels. Suitable drying techniques are well known to those skilled in the art and include the use of a molecular sieve or other inorganic desiccant and membrane drying processes.

In the stabilisation method of the fifth aspect of the present invention, typical and preferred amounts of the drug component and the propellant component and suitable, typical and preferred compositions for the propellant component are as discussed above for the pharmaceutical composition of the first aspect of the invention.

The drug component in the stabilisation method of the fifth aspect of the present invention may consist essentially of or consist entirely of salbutamol. By the term "consists essentially of", we mean that at least 98 weight %, more preferably at least 99 weight % and especially at least 99.9 weight % of the drug component consists of salbutamol. Alternatively, the drug component may additionally comprise at least one long acting muscarinic antagonist and/or at least one corticosteroid. When a long acting muscarinic antagonist and/or a corticosteroid are included, suitable and preferred long acting muscarinic antagonists and suitable and preferred corticosteroids are as described above for the pharmaceutical compositions of the second and third aspects of the present invention.

In one embodiment, the pharmaceutical composition in the fifth aspect of the present invention consists essentially of and more preferably consists entirely of the drug component and the propellant component as defined above. By the term "consists essentially of", we mean that at least 98 weight %, more preferably at least 99 weight % and especially at least 99.9 weight % of the pharmaceutical composition consists of the two components.

In an alternative embodiment, the pharmaceutical composition in the fifth aspect of the invention may contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In one preferred stabilisation method, the resulting pharmaceutical composition after storage at 40°C and 75 % relative humidity for 1 month will produce less than 3.0 % by weight and preferably less than 2.5 % by weight of impurities from the degradation of the salbutamol based on the total weight of the salbutamol and the impurities.

In another preferred stabilisation method in which the pharmaceutical composition also comprises at least one corticosteroid and/or at least one long acting muscarinic antagonist, the resulting pharmaceutical composition after storage at 40°C and 75 % relative humidity for 1 month will produce less than 3.0 % by weight and preferably less than 2.5 % by weight of impurities from the degradation of the salbutamol based on the total weight of the salbutamol and the impurities.

In yet another preferred stabilisation method, at least 90.0 % by weight, preferably at least 92.5 % by weight and more preferably at least 95.0 % by weight of the salbutamol that is contained originally in the pharmaceutical composition immediately following preparation will be present in the composition after storage at 40°C and 75 % relative humidity for 1 month.

In still another preferred stabilisation method in which the pharmaceutical composition also comprises at least one corticosteroid and/or at least one long acting muscarinic antagonist, at least 90.0 % by weight, preferably at least 92.5 % by weight and more preferably at least 95.0 % by weight of the salbutamol that is contained originally in the pharmaceutical composition immediately following preparation will be present in the composition after storage at 40°C and 75 % relative humidity for 1 month.

In a further preferred stabilisation method, at least 90.0 %, preferably at least 92.5 % and more preferably at least 95.0 % of the original pharmaceutical activity of the salbutamol is retained after storage at 40°C and 75 % relative humidity for 1 month.

In yet another preferred stabilisation method, the resulting pharmaceutical composition after storage at 50°C and 75 % relative humidity for 5 days will produce less than 1.0 % by weight, preferably less than 0.5 % by weight and more preferably less than 0.1 % by weight of impurities from the degradation of the salbutamol based on the total weight of the salbutamol and the impurities.

In another preferred stabilisation method in which the pharmaceutical composition also comprises at least one corticosteroid and/or at least one long acting muscarinic antagonist, the resulting pharmaceutical composition after storage at 50°C and 75 % relative humidity for 5 days will produce less than 1.0 % by weight, preferably less than 0.5 % by weight and more preferably less than 0.1 % by weight of impurities from the degradation of the salbutamol based on the total weight of the salbutamol and the impurities.

In yet another preferred stabilisation method, at least 96.0 % by weight, preferably at least 97.0 % by weight and more preferably at least 98.0 % by weight of the salbutamol that is contained originally in the pharmaceutical composition immediately following preparation will be present in the composition after storage at 50°C and 75 % relative humidity for 5 days.

In still another preferred stabilisation method in which the pharmaceutical composition also comprises at least one corticosteroid and/or at least one long acting muscarinic antagonist, at least 96.0 % by weight, preferably at least 97.0 % by weight and more preferably at least 98.0 % by weight of the salbutamol that is contained originally in the pharmaceutical composition immediately following preparation will be present in the composition after storage at 50°C and 75 % relative humidity for 5 days.

In a further preferred stabilisation method, at least 96.0 %, preferably at least 97.0 % and more preferably at least 98.0 % of the original pharmaceutical activity of the salbutamol is retained after storage at 50°C and 75 % relative humidity for 5 days.

One preferred pharmaceutical composition of the first, second, third and fourth aspects of the present invention will produce less than 3.0 % by weight and preferably less than 2.5 % by weight of total impurities from the degradation of the salbutamol after storage at 40°C and 75 % relative humidity for 1 month.

Another preferred pharmaceutical composition of the first, second, third and fourth aspects of the present invention will produce less than 1.0 % by weight, preferably less than 0.5 % by weight and more preferably less than 0.1 % by weight of impurities from the degradation of the salbutamol after storage at 50°C and 75 % relative humidity for 5 days.

The weight % of impurities indicated above are based on the total weight of the salbutamol and the impurities.

In a further preferred pharmaceutical composition of the first, second, third and fourth aspects of the present invention at least 90.0 % by weight, preferably at least 92.5 % by weight and more preferably at least 95.0 % by weight of the salbutamol that is contained originally in the pharmaceutical composition of the invention immediately following preparation will be present in the composition after storage at 40°C and 75 % relative humidity for 1 month.

In yet another preferred pharmaceutical composition of the first, second, third and fourth aspects of the present invention at least 90.0 %, preferably at least 92.5 % and more preferably at least 95.0 % of the original pharmaceutical activity of the salbutamol is retained after storage at 40°C and 75 % relative humidity for 1 month.

In a further preferred pharmaceutical composition of the first, second, third and fourth aspects of the present invention at least 96.0 % by weight, preferably at least 97.0 % by weight and more preferably at least 98.0 % by weight of the salbutamol that is contained originally in the pharmaceutical composition of the invention immediately following preparation will be present in the composition after storage at 50°C and 75 % relative humidity for 5 days.

In yet another preferred pharmaceutical composition of the first, second, third and fourth aspects of the present invention at least 96.0 %, preferably at least 97.0 % and more preferably at least 98.0 % of the original pharmaceutical activity of the salbutamol is retained after storage at 50°C and 75 % relative humidity for 5 days.

In referring to the storage of the pharmaceutical compositions in the above described stabilisation methods, we are referring, in particular, to the storage of those compositions in uncoated aluminium containers. Similarly, in referring to the storage of the above described pharmaceutical compositions, we are referring, in particular, to their storage in uncoated aluminium containers.

It has also been found that the use of a propellant comprising 1,1-difluoroethane (HFA-152a) in pharmaceutical compositions containing salbutamol and the propellant that are designed to be delivered using a metered dose inhaler can unexpectedly improve the aerosolization performance of the pharmaceutical composition after storage when that composition is delivered from the metered dose inhaler compared to the performance that is observed when HFA-134a is used as the propellant. In particular, the fine particle fraction of the salbutamol in the emitted dose after storage of the pharmaceutical composition at 50°C and 75 % relative humidity for 30 days typically comprises at least 40.0 weight %, preferably at least 42.5 weight % and more preferably at least 45.0 weight % of the emitted dose of the salbutamol.

Accordingly, in a sixth aspect of the present invention there is provided a method of improving the aerosolization performance after storage of a pharmaceutical composition comprising a propellant component and a drug component comprising salbutamol base, said method comprising using a propellant component at least 90 weight % of which is 1,1-difluoroethane (HFA-152a).

The pharmaceutical composition in the method of the sixth aspect of the present invention may be a suspension or a solution.

In a preferred embodiment of the sixth aspect of the present invention there is provided a method of improving the aerosolization performance after storage of a pharmaceutical composition comprising a propellant component and a drug component comprising salbutamol, said method comprising using a propellant component comprising 1,1-difluoroethane (HFA-152a) and providing a pharmaceutical composition which when delivered from a metered dose inhaler yields a fine particle fraction of the salbutamol which is at least 40.0 weight %, preferably at least 42.5 weight % and more preferably at least 45.0 weight % of the emitted dose of the salbutamol even after storage of the pharmaceutical composition at 50°C and 75 % relative humidity for 30 days.

Increasing the fine particle fraction of the emitted dose after long term storage is highly beneficial. It is the fine drug particles that are able to penetrate into the deep bronchiole passages and the alveolar passages of the lung to maximise relief from the effects of an asthma attack or COPD. Thus, retaining a high fine particle fraction after storage means that the user of the MDI should still receive a medically satisfactory dose of the drug even though a significant period of time has elapsed since the pharmaceutical composition was first manufactured.

The fine particle fraction is a widely recognised term in the art. It is a measure of the mass fraction of emitted aerosol particles having a diameter below 5 µm which is generally accepted as being the most desirable particle size range for effective alveolar drug delivery.

In the method of the sixth aspect of the present invention, typical and preferred amounts of the drug component and the propellant component and suitable, typical and preferred compositions for the propellant component are as discussed above for the pharmaceutical composition of the first aspect of the invention.

The drug component in the method of the sixth aspect of the present invention may consist essentially of or consist entirely of the salbutamol. By the term "consists essentially of", we mean that at least 98 weight %, more preferably at least 99 weight % and especially at least 99.9 weight % of the drug component consists of salbutamol. Alternatively, the drug component may additionally comprise at least one long acting muscarinic antagonist and/or at least one corticosteroid. When a long acting muscarinic antagonist and/or corticosteroid are included, suitable and preferred long acting muscarinic antagonists and suitable and preferred corticosteroids are as described above for the pharmaceutical compositions of the second and third aspects of the present invention.

In one embodiment, the pharmaceutical composition in the sixth aspect of the present invention consists essentially of and more preferably consists entirely of the drug component and the propellant component as defined above. By the term "consists essentially of", we mean that at least 98 weight %, more preferably at least 99 weight % and especially at least 99.9 weight % of the pharmaceutical composition consists of the two components.

In an alternative embodiment, the pharmaceutical composition in the sixth aspect of the invention may contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

The pharmaceutical compositions of the invention find particular utility in the delivery of the salbutamol, and where included the corticosteroid and long acting muscarinic antagonist compounds, from a pressurised aerosol container, e.g. using a metered dose inhaler (MDI). For this application, the pharmaceutical compositions are contained in the pressurised aerosol container and the HFA-152a propellant functions to deliver the drug as a fine aerosol spray.

The pharmaceutical compositions of the invention may comprise one or more other additives of the type that are conventionally used in drug formulations for pressurised MDIs, such as valve lubricants. Where other additives are included in the pharmaceutical compositions, they are normally used in amounts that are conventional in the art.

The pharmaceutical compositions of the invention are normally stored in a pressurised container or canister which is to be used in association with a medication delivery device. When so stored, the pharmaceutical compositions are normally a liquid. In a preferred embodiment, the pressurised container is designed for use in a metered dose inhaler (MDI). In a particularly preferred embodiment, the pressurised container is a coated aluminium can or an uncoated aluminium can, especially the latter.

Accordingly, a seventh aspect of the present invention provides a pressurised container holding the pharmaceutical composition of the first, second, third or fourth aspect of the present invention. In an eighth aspect, the present invention provides a medication delivery device, especially a metered dose inhaler, having a pressurised container holding the pharmaceutical composition of the first, second, third or fourth aspect of the present invention.

The metered dose inhaler typically comprises a nozzle and valve assembly that is crimped to a container holding the pharmaceutical composition to be dispensed. An elastomeric gasket is used to provide a seal between the container and the nozzle/valve assembly. Preferred elastomeric gasket materials are EPDM, chlorobutyl, bromobutyl and cycloolefin copolymer rubbers as these can exhibit good compatibility with HFA-152a and also provide a good barrier to prevent or limit HFA-152a permeating from the container.

The pharmaceutical compositions of the present invention are for use in medicine for treating a patient suffering or likely to suffer from a respiratory disorder and especially asthma or a chronic obstructive pulmonary disease.

Accordingly, the present invention also provides a method for treating a patient suffering or likely to suffer from a respiratory disorder, especially asthma or a chronic obstructive pulmonary disease, which comprises administering to the patient a therapeutically or prophylactically effective amount of a pharmaceutical composition as discussed above. The pharmaceutical composition is preferably delivered to the patient using a MDI.

The pharmaceutical compositions of the invention can be prepared and the MDI devices filled using techniques that are standard in the art, such as pressure filling and cold filling. For example, the pharmaceutical compositions can be prepared by a simple blending operation in which the salbutamol, optionally the at least one corticosteroid and/or the at least one long acting muscarinic antagonist, optionally the surfactant component, optionally the polar excipient, and the HFA-152a-containing propellant are mixed together in the required proportions in a suitable mixing vessel. Mixing can be promoted by stirring as is common in the art. Conveniently, the HFA-152a-containing propellant is liquefied to aid mixing. If the pharmaceutical composition is made in a separate mixing vessel, it can then be transferred to pressurised containers for storage, such as pressurised containers that are used as part of medication delivery devices and especially MDIs.

The pharmaceutical compositions of the invention can also be prepared within the confines of a pressurised container, such as an aerosol canister or vial, from which the compositions are ultimately released as an aerosol spray using a medication delivery device, such as a MDI. In this method, a weighed amount of the salbutamol and optionally the at least one corticosteroid and/or the at least one long acting muscarinic antagonist, is introduced into the open container. A valve is then crimped onto the container and the HFA-152a-containing propellant component, in liquid form, introduced through the valve into the container under pressure, optionally after first evacuating the container through the valve. The surfactant component and/or polar excipient, if included, can be mixed with the drug(s) or, alternatively, introduced into the container after the valve has been fitted, either alone or as a premix with the propellant component. The whole mixture can then be treated to disperse the drugs in the propellant/propellant mixture, e.g. by vigorous shaking or using an ultrasonic bath. Suitable containers may be made of plastics, metal, e.g. aluminium, or glass. Preferred containers are made of metal, especially aluminium which may be coated or uncoated. Uncoated aluminium containers are especially preferred.

The container may be filled with enough of the pharmaceutical composition to provide for a plurality of dosages. The pressurized aerosol canisters that are used in MDIs typically contain 50 to 150 individual dosages.

The present invention also provides a method of reducing the global warming potential (GWP) of a pharmaceutical composition comprising a drug component comprising salbutamol base and a propellant component, said method comprising using a propellant component comprising 1,1-difluoroethane (HFA-152a). This method is applicable to the preparation of all the pharmaceutical compositions disclosed herein in all their aspects and embodiments.

Preferably, at least 90 weight %, more preferably at least 95 weight % and still more preferably at least 99 weight % of the propellant component used is HFA-152a. In an especially preferred embodiment, the propellant component used is entirely HFA-152a.

The propellant component that is used will preferably have a global warming potential (GWP) of less than 250, more preferably less than 200 and still more preferably less than 150.

The present invention is now illustrated but not limited by the following examples.

### Example 1

A number of experiments were conducted to investigate the *in vitro* aerosolization performance of pharmaceutical formulations of salbutamol base delivered from a metered dose inhaler (MDI) using either HFA-134a or HFA-152a as the propellant after initial preparation and after storing under stress storage conditions.

Pharmaceutical formulations of salbutamol were prepared in either HFA-134a or HFA-152a (Mexichem, UK) and 10 % w/w ethanol. The salbutamol (30 g) was suspended in the ethanol (970 g) and homogenized using a Silverson High-Shear homogenizer. 1 g of the resultant slurry was then weighed into standard uncoated 14 ml aluminium canisters (C128, Presspart, Blackburn, UK). The canisters were then crimped with a 50 µL valve (Bespak, Kings Lynn, UK) following which the propellant was filled into the canisters through the valve using a manual Pamasol crimper/filler (Pamasol, Switzerland). Finally, the canisters were sonicated for 20 minutes to aid dispersion of the drug in the suspension. The nominal does of salbutamol was 120 µg.

The *in vitro* aerosolization performance of the formulations was tested immediately after preparation (time t = zero) with a Next Generation Impactor using the method described below. The formulations were then stored under stress storage conditions (valve down) at 50°C and 75 % relative humidity for a total of 30 days. The *in vitro* aerosolization performance of the pharmaceutical formulations was tested as before after 5, 10 and 30 days under the stress storage conditions with a Next Generation Impactor using the method described below.

The Next Generation Impactor (NGI, Copley Scientific, Nottingham UK) was connected to a vacuum pump (GE Motors, NJ, USA). Prior to testing, the cups of the NGI system were coated with 1 % v/v silicone oil in hexane to eliminate particle bounce. For each experiment, three actuations of the valve were discharged into the NGI at 30 L.min⁻¹ as per pharmacopeia guidelines. Following aerosolization, the NGI apparatus was dismantled and the actuator and each part of the NGI was washed down into known volumes of the selected HPLC mobile phase (see below). The mass of drug deposited on each part of the NGI was determined by HPLC using the methodology described below. This protocol was repeated three times for each canister, following which, the fine particle dose (FPD) and fine particle fraction of the emitted dose (FPF_{ED}) were determined. The results are shown in Tables 3 and 4 below.

High performance liquid chromatography (HPLC) was used to determine drug content following aerosolization studies (see below). A Hypersil BDS C18 column (Fisher, UK, 5µm particle size, 250 mm × 4.6 mm internal diameter) was used for the analysis. The column was coupled to a UV detector (Agilent 1200) operating at a wavelength of 240 nm. The chromatographic conditions are shown in Table 1 below.

**Table 1**

| **Drug** | **Pump Flow Rate (ml.min⁻¹)** | **Mobile Phase** | **UV Wavelength (nm)** | **Column Temperature (°C)** |
|---|---|---|---|---|
| Salbutamol base | 0.4 | Solution A: 10 mM pentafluoropropionic acid in water | 240 | 30 |
| | | Solution B: Water/acetonitrile (1:1) | | |

The composition of the mobile phase was varied as shown in Table 2 below.

**Table 2**

| **Time (minutes)** | **Volume % Solution A** | **Volume % Solution B** |
|---|---|---|
| 0.00 | 85 | 15 |
| 9.00 | 35 | 65 |
| 11.50 | 5 | 95 |
| 12.50 | 5 | 95 |
| 12.51 | 85 | 15 |
| 16.00 | 85 | 15 |

The results of the aerosolization studies are shown in Tables 3 and 4 below.

**Table 3. In vitro aerosolization performance of salbutamol base delivered from a MDI when formulated with ethanol and HFA-134a as the propellant at time t = 0 and after storage (valve down) for 5, 10 and 30 days at 50°C and 75 % relative humidity as characterised by the emitted dose, fine particle dose, fine particle fraction of the emitted dose (FPF_{ED} %), mass median aerodynamic diameter (MMAD) and geometric standard deviation (GSD).**

| | T=0 | T=5 days @ 50°C/75% RH | T=10 days @ 50°C/75% RH | T=30 days @ 50°C/75% RH |
|---|---|---|---|---|
| Emitted Dose (µg) | 94.07 | 94.33 | 89.92 | 76.20 |
| MMAD (µm) | 2.36 | 2.42 | 2.77 | 4.94 |
| GSD | 2.12 | 2.35 | 2.45 | 2.10 |
| Fine Particle Dose (µg) | 50.53 | 44.96 | 39.77 | 26.89 |
| FPF_{ED} % | 53.72 | 47.66 | 44.23 | 35.29 |

**Table 4. In vitro aerosolization performance of salbutamol base delivered from a MDI when formulated with ethanol and HFA-152a as the propellant at time t = 0 and after storage (valve down) for 5, 10 and 30 days at 50°C and 75 % relative humidity as characterised by the emitted dose, fine particle dose, fine particle fraction of the emitted dose (FPF_{ED} %), mass median aerodynamic diameter (MMAD) and geometric standard deviation (GSD).**

| | T=0 | T=5 days @ 50°C/75% RH | T=10 days @ 50°C/75% RH | T=30 days @ 50°C/75% RH |
|---|---|---|---|---|
| Emitted Dose (µg) | 98.33 | 94.84 | 90.51 | 92.92 |
| MMAD (µm) | 2.37 | 2.33 | 2.42 | 3.75 |
| GSD | 2.03 | 2.11 | 2.15 | 1.85 |
| Fine particle Dose (µg) | 56.04 | 50.14 | 43.12 | 43.22 |
| FPF_{ED} % | 56.99 | 52.87 | 47.64 | 46.51 |

It is evident from the data presented in Tables 3 and 4 above that the emitted dose and the fine particle fraction of the emitted dose upon aerosolization were significantly better after storage under stress storage conditions when HFA-152a was used as the propellant. This is advantageous as it indicates that salbutamol base formulations with HFA-152a will maintain better aerosolization performance under normal storage conditions than when HFA-134a is used as the propellant.

### Example 2

The stability of salbutamol in HFA-134a and HFA-152a was investigated at time zero (T=0) and after storage, valve down, in uncoated aluminium canisters for 1 month (T=1M) and 3 months (T=3M) at 40°C and 75% relative humidity (RH) and for 5, 10, 15 and 30 days at 50°C and 75% relative humidity (RH).

The drug formulations were prepared and analysed using HPLC in accordance with the procedures described in Example 1.

The results of investigating the chemical stability of the salbutamol drug formulations in HFA-152a and HFA-227ea in uncoated aluminium cans are shown, respectively, in Tables 5 and 6 below.

**Table 5. Chemical stability of salbutamol base in HFA-134a in uncoated aluminium canisters based on percentage assay and total impurities upon storage at T=0, T=1 M @ 40°C/75 % RH, T=3M @ 40°C/75 % RH, T=5 days at 50°C/75 % RH, T=10 days at 50°C/75 % RH, T=15 days at 50°C/75 % RH and T=30 days at 50°C/75 % RH.**

| Time | % Assay (LC) | % Total Impurities |
|---|---|---|
| Initial time T = 0 | 99.8 | None detectable |
| T = 5 days @ 50/75 | 92.9 | 3.2 |
| T = 10 days @ 50/75 | 88.9 | 4.8 |
| T = 15 days @ 50/75 | 83.8 | 5.5 |
| T = 30 days @ 50/75 | 73.8 | 8.3 |
| T = 1M @ 40/75 | 87.5 | 4.8 |
| T = 3M @ 40/75 | 68.9 | >10 |

**Table 6. Chemical stability of salbutamol base in HFA-152a in uncoated aluminium canisters based on percentage assay and total impurities upon storage at T=0, T=1M @ 40°C/75 % RH, T=3M @ 40°C/75 % RH, T=5 days at 50°C/75 % RH, T=10 days at 50°C/75 % RH, T=15 days at 50°C/75 % RH and T=30 days at 50°C/75 % RH.**

| Time | % Assay (LC) | % Total Impurities |
|---|---|---|
| Initial time T = 0 | 101.5 | None detectable |
| T = 5 days @ 50/75 | 98.5 | None detectable |
| T = 10 days @ 50/75 | 96.8 | 1.2 |
| T = 15 days @ 50/75 | 93.2 | 2.8 |
| T = 30 days @ 50/75 | 89.4 | 3.7 |
| T = 1M @ 40/75 | 95.5 | 1.8 |
| T = 3M @ 40/75 | 78.9 | 8.2 |

It can be seen from the data in Tables 5 and 6 above that salbutamol exhibits superior chemical stability when blended with HFA-152a as the aerosolization propellant rather than HFA-134a.

## Claims

1. A pharmaceutical composition comprising:
(i) a drug component comprising salbutamol base; and
(ii) a propellant component at least 90 weight % of which is 1,1-difluoroethane (HFA-152a).

2. The pharmaceutical composition of claim 1, wherein the composition contains less than 1000 ppm, preferably less than 500 ppm, more preferably less than 100 ppm, still more preferably less than 50 ppm, particularly less than 10 ppm and especially less than 5 ppm of water based on the total weight of the pharmaceutical composition.

3. The pharmaceutical composition of claim 2, wherein the composition contains greater than 0.5 ppm, e.g. greater than 1 ppm, of water based on the total weight of the pharmaceutical composition.

4. The pharmaceutical composition of any one of the preceding claims, wherein the drug component additionally comprises at least one long acting muscarinic antagonist (LAMA).

5. The pharmaceutical composition of claim 4, wherein the at least one long acting muscarinic antagonist is selected from the group consisting of umeclidinium, ipratropium, tiotropium, aclidinium and the pharmaceutically acceptable salts thereof.

6. The pharmaceutical composition of claim 4, wherein the at least one long acting muscarinic antagonist is a pharmaceutically acceptable salt of glycopyrrolate, especially glycopyrronium bromide.

7. The pharmaceutical composition of any one of the preceding claims, wherein the drug component additionally comprises at least one corticosteroid.

8. The pharmaceutical composition of claim 7, wherein the at least one corticosteroid is selected from the group consisting of budesonide, mometasone, beclomethasone, fluticasone and the pharmaceutically acceptable salts and esters thereof.

9. The pharmaceutical composition of any one of the preceding claims, wherein the drug component comprises from 0.01 to 2.5 weight % of the total weight of the pharmaceutical composition and the propellant component comprises from 80.0 to 99.99 weight % of the total weight of the pharmaceutical composition.

10. The pharmaceutical composition of any one of the preceding claims, wherein at least 95 weight % and preferably at least 99 weight % of the propellant component is 1,1-difluoroethane (HFA-152a).

11. The pharmaceutical composition of any one of the preceding claims further comprising a surfactant component comprising at least one surfactant compound, preferably at least one surfactant compound selected from polyvinylpyrrolidone, polyethylene glycol surfactants, oleic acid and lecithin.

12. The pharmaceutical composition of any one of the preceding claims further comprising a polar excipient, preferably ethanol.

13. The pharmaceutical composition of any one of the preceding claims which after storage in uncoated aluminium containers at 40°C and 75 % relative humidity for 1 month will produce less than 3.0 % by weight and preferably less than 2.5 % by weight of impurities from the degradation of the salbutamol based on the total weight of the salbutamol and the impurities and wherein at least 90.0 % by weight, preferably at least 92.5 % by weight and more preferably at least 95.0 % by weight of the salbutamol that is contained originally in the pharmaceutical composition immediately following preparation will be present in the composition after storage in uncoated aluminium containers at 40°C and 75 % relative humidity for 1 month.

14. The pharmaceutical composition of any one of the preceding claims which when delivered from a metered dose inhaler yields a fine particle fraction of the salbutamol which is at least 40.0 weight %, preferably at least 42.5 weight % and more preferably at least 45.0 weight % of the emitted dose of the salbutamol even after storage of the pharmaceutical composition at 50°C and 75 % relative humidity for 30 days.

15. The pharmaceutical composition of any one of the preceding claims in the form of a suspension.

16. The pharmaceutical composition of any one of claims 1 to 14 in the form of a solution.

17. A metered dose inhaler (MDI) fitted with a sealed and pressurized aerosol container that contains a pharmaceutical composition as claimed in any one of claims 1 to 16.

18. A method of improving the stability of a pharmaceutical composition comprising a propellant component and a drug component comprising salbutamol base, said method comprising using a propellant component at least 90 weight % of which is 1,1-difluoroethane (HFA-152a).

19. A method of improving the aerosolization performance after storage of a pharmaceutical composition comprising a propellant component and a drug component comprising salbutamol base, said method comprising using a propellant component at least 90 weight % of which is 1,1-difluoroethane (HFA-152a).

20. The method of claim 18 or 19, wherein the pharmaceutical composition is a composition as claimed in any one of claims 2 to 16.

## Patentansprüche

1. Pharmazeutische Zusammensetzung, die Folgendes umfasst:
(i) eine Arzneimittelkomponente, die eine Salbutamol-Basis umfasst; und
(ii) eine Treibmittelkomponente, von der wenigstens 90 Gew.-% 1,1-Difluorethan (HFA-152a) sind.

2. Pharmazeutische Zusammensetzung nach Anspruch 1, wobei die Zusammensetzung weniger als 1000 ppm, bevorzugt weniger als 500 ppm, stärker bevorzugt weniger als 100 ppm, noch stärker bevorzugt weniger als 50 ppm, insbesondere weniger als 10 ppm und speziell weniger als 5 ppm Wasser basierend auf dem Gesamtgewicht der pharmazeutischen Zusammensetzung enthält.

3. Pharmazeutische Zusammensetzung nach Anspruch 2, wobei die Zusammensetzung über 0,5 ppm, z. B. über 1 ppm, Wasser basierend auf dem Gesamtgewicht der pharmazeutischen Zusammensetzung enthält.

4. Pharmazeutische Zusammensetzung nach einem der vorhergehenden Ansprüche, wobei die Arzneimittelkomponente zusätzlich wenigstens einen langwirkenden Muskarinantagonisten (long acting muscarinic antagonist - LAMA) umfasst.

5. Pharmazeutische Zusammensetzung nach Anspruch 4, wobei der wenigstens eine langwirkende Muskarinantagonist aus der Gruppe ausgewählt ist, die aus Umeclidinium, Ipratropium, Tiotropium, Aclidinium und den pharmazeutisch unbedenklichen Salzen davon besteht.

6. Pharmazeutische Zusammensetzung nach Anspruch 4, wobei der wenigstens eine langwirkende Muskarinantagonist ein pharmazeutisch unbedenkliches Salz von Glycopyrrolat, speziell Glycopyrroniumbromid, ist.

7. Pharmazeutische Zusammensetzung nach einem der vorhergehenden Ansprüche, wobei die Arzneimittelkomponente zusätzlich wenigstens ein Corticosteroid umfasst.

8. Pharmazeutische Zusammensetzung nach Anspruch 7, wobei das wenigstens eine Corticosteroid aus der Gruppe ausgewählt ist, die aus Budesonid, Mometason, Beclomethason, Fluticason und den pharmazeutisch unbedenklichen Salzen und

9. Pharmazeutische Zusammensetzung nach einem der vorhergehenden Ansprüche, wobei die Arzneimittelkomponente von 0,01 bis 2,5 Gew.-% des Gesamtgewichts der pharmazeutischen Zusammensetzung umfasst und die Treibmittelkomponente von 80,0 bis 99,99 Gew.-% des Gesamtgewichts der pharmazeutischen Zusammensetzung umfasst.

10. Pharmazeutische Zusammensetzung nach einem der vorhergehenden Ansprüche, wobei wenigstens 95 Gew.-% und bevorzugt wenigstens 99 Gew.-% der Treibmittelkomponente 1,1-Difluorethan (HFA-152a) sind.

11. Pharmazeutische Zusammensetzung nach einem der vorhergehenden Ansprüche, die ferner eine Tensidkomponente umfasst, die wenigstens eine Tensidverbindung, bevorzugt wenigstens eine Tensidverbindung umfasst, die aus Polyvinylpyrrolidon, Polyethylenglycoltensiden, Ölsäure und Lecithin ausgewählt ist.

12. Pharmazeutische Zusammensetzung nach einem der vorhergehenden Ansprüche, die ferner einen polaren Arzneistoffträger, bevorzugt Ethanol, umfasst.

13. Pharmazeutische Zusammensetzung nach einem der vorhergehenden Ansprüche, die nach Lagerung in unbeschichteten Aluminiumbehältern 1 Monat lang bei 40 °C und 75 % relativer Luftfeuchtigkeit weniger als 3,0 % Gew.-% und bevorzugt weniger als 2,5 % Gew.-% an Verunreinigungen von dem Abbau des Salbutamols basierend auf dem Gesamtgewicht des Salbutamols und den Verunreinigungen produzieren wird und wobei wenigstens 90,0 Gew.-%, bevorzugt wenigstens 92,5 Gew.-% und stärker bevorzugt wenigstens 95,0 Gew.-%, des Salbutamols, das ursprünglich in der pharmazeutischen Zusammensetzung enthalten ist, sofort anschließend an eine Herstellung in der Zusammensetzung nach Lagerung in unbeschichteten Aluminiumbehältern 1 Monat lang bei 40 °C und 75 % bezogen auf Luftfeuchtigkeit vorhanden sein wird.

14. Pharmazeutische Zusammensetzung nach einem der vorhergehenden Ansprüche, die, wenn sie von einem Inhalator für eine gemessene Dosis abgegeben wird, eine Feinpartikelfraktion des Salbutamol ergibt, die wenigstens 40,0 Gew.-%, bevorzugt wenigstens 42,5 Gew.-% und stärker bevorzugt wenigstens 45,0 Gew.-%, der emittierten Dosis des Salbutamol sogar nach Lagerung der pharmazeutischen Zusammensetzung 30 Tage lang bei 50 °C und 75 % relativer Luftfeuchtigkeit beträgt.

15. Pharmazeutische Zusammensetzung nach einem der vorhergehenden Ansprüche in der Form einer Suspension.

16. Pharmazeutische Zusammensetzung nach einem der Ansprüche 1 bis 14 in der Form einer Lösung.

17. Inhalator für eine gemessene Dosis (metered dose inhaler- MDI), der mit einem versiegelten und unter Druck stehenden Aerosolbehälter ausgerüstet ist, der eine pharmazeutische Zusammensetzung nach einem der Ansprüche 1 bis 16 enthält.

18. Verfahren zum Verbessern der Stabilität einer pharmazeutischen Zusammensetzung, die eine Treibmittelkomponente und eine Arzneimittelkomponente umfasst, die eine Salbutamol-Basis umfasst, wobei das Verfahren ein Verwenden einer Treibmittelkomponente umfasst, von der wenigstens 90 Gew.-% 1,1-Difluorethan (HFA-152a) sind.

19. Verfahren zum Verbessern der Aerosolisierungsleistung nach Lagerung einer pharmazeutischen Zusammensetzung, die eine Treibmittelkomponente und eine Arzneimittelkomponente umfasst, die eine Salbutamol-Basis umfasst, wobei das Verfahren das Verwenden einer Treibmittelkomponente umfasst, von der wenigstens 90 Gew.-% 1,1-Difluorethan (HFA-152a) sind.

20. Verfahren nach einem der Ansprüche 18 oder 19, wobei die pharmazeutische Zusammensetzung eine Zusammensetzung nach einem der Ansprüche 2 bis 16 ist.

## Revendications

1. Composition pharmaceutique comprenant :
(i) un composant médicamenteux comprenant une base de salbutamol ; et
(ii) un composant propulseur dont au moins 90 % en poids est du 1,1-difluoroéthane (HFA-152a).

2. Composition pharmaceutique selon la revendication 1, la composition contenant moins de 1 000 ppm, de préférence moins de 500 ppm, plus préférablement moins de 100 ppm, encore plus préférablement moins de 50 ppm, particulièrement moins de 10 ppm et en particulier moins de 5 ppm d'eau sur la base du poids total de la composition pharmaceutique.

3. Composition pharmaceutique selon la revendication 2, la composition contenant plus de 0,5 ppm, par exemple plus de 1 ppm, d'eau sur la base du poids total de la composition pharmaceutique.

4. Composition pharmaceutique selon l'une quelconque des revendications précédentes, le composant médicamenteux comprenant en outre au moins un antagoniste muscarinique à action prolongée (LAMA).

5. Composition pharmaceutique selon la revendication 4, l'au moins un antagoniste muscarinique à action prolongée étant choisi dans le groupe constitué d'umeclidinium, d'ipratropium, de tiotropium, d'aclidinium et leurs sels pharmaceutiquement acceptables.

6. Composition pharmaceutique selon la revendication 4, l'au moins un antagoniste muscarinique à action prolongée étant un sel pharmaceutiquement acceptable de glycopyrrolate, en particulier de bromure de glycopyrronium.

7. Composition pharmaceutique selon l'une quelconque des revendications précédentes, le composant médicamenteux comprenant d'autre part au moins un corticostéroïde.

8. Composition pharmaceutique selon la revendication 7, l'au moins un corticostéroïde étant choisi dans le groupe constitué de budésonide, de mométasone, de béclométhasone, de fluticasone et de leurs sels et esters pharmaceutiquement

9. Composition pharmaceutique selon l'une quelconque des revendications précédentes, le composant médicamenteux comprenant de 0,01 à 2,5 % en poids du poids total de la composition pharmaceutique et le composant propulseur comprenant de 80,0 à 99,99 % en poids du poids total de la composition pharmaceutique.

10. Composition pharmaceutique selon l'une quelconque des revendications précédentes, au moins 95 % en poids et de préférence au moins 99 % en poids du composant propulseur étant du 1,1-difluoroéthane (HFA-152a).

11. Composition pharmaceutique selon l'une quelconque des revendications précédentes comprenant en outre un composant surfactant comprenant au moins un composé surfactant, de préférence au moins un composé surfactant choisi parmi la polyvinylpyrrolidone, les surfactants de polyéthylène glycol, l'acide oléique et la lécithine.

12. Composition pharmaceutique selon l'une quelconque des revendications précédentes, comprenant en outre un excipient polaire, de préférence de l'éthanol.

13. Composition pharmaceutique selon l'une quelconque des revendications précédentes, qui après un stockage dans des récipients en aluminium non revêtus à 40 °C et 75 % d'humidité relative pendant 1 mois produira moins de 3,0 % en poids et de préférence moins de 2,5 % en poids d'impuretés de la dégradation du salbutamol sur la base du poids total du salbutamol et des impuretés et, au moins 90,0 % en poids, de préférence au moins 92,5 % en poids et plus préférablement au moins 95,0 % en poids du salbutamol qui est contenu à l'origine dans la composition pharmaceutique immédiatement après sa préparation étant présent dans la composition après stockage dans des récipients en aluminium non revêtus à 40 °C et à 75 % d'humidité relative pendant 1 mois.

14. Composition pharmaceutique selon l'une quelconque des revendications précédentes, qui, lorsqu'elle est administrée à partir d'un aérosol-doseur, donne une fraction de particules fines du salbutamol qui est d'au moins 40,0 % en poids, de préférence d'au moins 42,5 % en poids et plus préférablement d'au moins 45,0 % en poids de la dose émise du salbutamol même après un stockage de la composition pharmaceutique à 50 °C et 75 % d'humidité relative pendant 30 jours.

15. Composition pharmaceutique selon l'une quelconque des revendications précédentes sous la forme d'une suspension.

16. Composition pharmaceutique selon l'une quelconque des revendications 1 à 14 sous la forme d'une solution.

17. Aérosol-doseur (MDI) équipé d'un récipient aérosol étanche et sous pression qui contient une composition pharmaceutique selon l'une quelconque des revendications 1 à 16.

18. Procédé d'amélioration de la stabilité d'une composition pharmaceutique comprenant un composant propulseur et un composant médicamenteux comprenant une base de salbutamol, ledit procédé comprenant l'utilisation d'un composant propulseur dont au moins 90 % en poids est du 1,1-difluoroéthane (HFA-152a).

19. Procédé d'amélioration de la performance d'aérosolisation après le stockage d'une composition pharmaceutique comprenant un composant propulseur et un composant médicamenteux comprenant une base de salbutamol, ledit procédé comprenant l'utilisation d'un composant propulseur dont au moins 90 % en poids est du 1,1-difluoroéthane (HFA-152a).

20. Procédé selon l'une quelconque des revendications 18 à 19, la composition pharmaceutique étant une composition selon l'une quelconque des revendications 2 à 16.
